# FASCICULE DE BREVET EUROPEEN

(11) **EP 1 758 588 B1**
(45) Date de publication et mention de la délivrance du brevet: **24.11.2010**
(21) Numéro de dépôt: 05777135.4
(22) Date de dépôt: 15.06.2005
(51) Int. Cl.: A61K 31/573, A61K 31/59, A61P 17/06

(54) **COMPOSITION PHARMACEUTIQUE COMPRENANT UN ONGUENT OLEAGINEUX ET DEUX PRINCIPES ACTIFS SOLUBILISES.**
PHARMAZEUTISCHE ZUSAMMENSETZUNG MIT EINER SALBE UND ZWEI AUFGELÖSTEN WIRKSTOFFEN
PHARMACEUTICAL COMPOSITION COMPRISING AN OINTMENT AND TWO SOLUBILIZED ACTIVE PRINCIPLES

(30) Priorité: 17.06.2004 FR 0406609
(43) Date de publication de la demande: 07.03.2007
(73) Titulaire: Galderma S.A., 6330 Cham (CH)
(72) Inventeur: ZANUTTO, Leslie, F-43230 Paulhaguet (FR); ORSONI, Sandrine, F-06210 Mandelieu (FR); BARTHEZ, Nathalie, F-06300 Nice (FR)
(74) Mandataire: Allab, Myriam
(86) Numéro de dépôt international: PCT/FR2005/001493
(87) Numéro de publication internationale: WO 2006/005842

(56) Documents cités:
- WO-A-00/64450
- WO-A-02/34235
- WO-A-2004/054588
- LAHFA M ET AL: "CALCITRIOL OINTMENT AND CLOBETASOL PROPIONATE CREAM: A NEW REGIMEN FOR THE TREATMENT OF PLAQUE PSORIASIS" EUROPEAN JOURNAL OF DERMATOLOGY, XX, XX, vol. 13, no. 3, mai 2003 (2003-05), pages 261-265, XP009016887 ISSN: 1167-1122
- ANONYMOUS: "Dovobet Oinment" INTERNET ARTICLE, [Online] 1 novembre 2004 (2004-11-01), XP002314150 Extrait de l'Internet: URL:http://emc.medicines.org.uk/emc/assets /c/html/displaydoc.asp?documentid=8851#COM POSITION> [extrait le 2005-01-19]
- LAMBA S ET AL: "Combination therapy with vitamin D analogues" BRITISH JOURNAL OF DERMATOLOGY, XX, XX, vol. 144, no. Supplement 58, avril 2001 (2001-04), pages 27-32, XP002253887 ISSN: 0007-0963
- KRAGBALLE K ET AL: "Efficacy of once-daily treatment regimens with calcipotriol/betamethasone dipropionate ointment and calcipotriol ointment in psoriasis vulgaris." THE BRITISH JOURNAL OF DERMATOLOGY. JUN 2004, vol. 150, no. 6, juin 2004 (2004-06), pages 1167-1173, XP001205154 ISSN: 0007-0963

## Description

La présente invention concerne le domaine de la formulation de principes actifs en vue d'une application pharmaceutique topique.

La présente invention se rapporte plus particulièrement à une composition pharmaceutique stable, anhydre comprenant un onguent oléagineux et à titre de principes actifs du calcitriol et du 17-propionate de clobétasol, à son procédé de préparation et à son utilisation pour le traitement topique du psoriasis et autres désordres cutanés.

Le calcitriol est un analogue de la vitamine D et est notamment utilisé pour réguler le taux de calcium dans l'organisme. La vitamine D et ses dérivés sont généralement utilisés en dermatologie dans le traitement du psoriasis car ils limitent la production excessive de cellules cutanées sur les surfaces atteintes et possèdent des avantages avérés pour le traitement de cette affection qui se caractérise notamment par la présence de lésions épaisses, squameuses et sèches.

Le 17-propionate de clobetasol est un corticostéroïde. Le mécanisme d'action des corticostéroïdes est attribué à leur inhibition des processus inflammatoires (Lange K et al, Skin Pharmacol Appl Skin Physiol 13(2) :93-103 (2000)).

Le document US 4,610,978 décrit des compositions pour application topique dans le traitement des maladies dermatologiques telles que par exemple le psoriasis, comprenant du calcitriol. Ces compositions peuvent en outre contenir un corticostéroïde tel que par exemple l'hydrocortisone ou la dexaméthasone. Les documents WO 00/64450 et WO 02/34235 décrivent des compositions pharmaceutiques pour usage dermique contenant de la vitamine D ou un analogue de la vitamine D et un corticostéroïde. Les exemples donnés concernent plus particulièrement des compositions comprenant du calcipotriol (analogue de la vitamine D) et de la bétaméthasone (corticostéroïde). La comparaison des mesures de l'efficacité sur des patients atteints de psoriasis d'une composition comprenant du calcipotriol seul, du bétaméthasone seul ou l'association des deux actifs montrent que l'effet obtenu par l'association correspond à un effet additif. Ainsi au regard de ce document, l'homme du métier ne pouvait nullement imaginer que l'association d'un analogue de la vitamine D avec un corticostéroide puisse présenter un effet synergique. En outre ce document ne décrit pas spécifiquement l'association de propionate de clobétasol avec du calcitriol.

Dans le document FR 2 848 454, la Demanderesse a découvert que l'association du calcitriol avec du propionate de clobétasol permettait d'obtenir un effet synergique dans le traitement de certaines affections dermatologiques telles que le psoriasis, la dermatite atopique, la dermatite de contact et la dermatite séborrhéique.

Cependant, l'association dans une même composition pharmaceutique de calcitriol avec du propionate de clobétasol n'est pas sans poser quelques problèmes. En effet, le calcitriol est instable dans les milieux aqueux et plus particulièrement aux pH acides, alors que le 17-propionate de clobétasol est instable dans un environnement basique.

En conséquence, il convient de formuler ces principes actifs dans des compositions de type anhydre. Les compositions anhydres disponibles actuellement, permettant la formulation de principes actifs sensibles à l'eau, tout en leur assurant une bonne stabilité chimique, sont généralement des compositions de type onguent. Ces compositions de type onguent sont constituées principalement de vaseline, d'huile minérale et/ou d'huile végétale. Cependant, les traitements actuellement sur le marché contiennent soit un fort pourcentage de vaseline pour favoriser l'occlusivité et la pénétration de l'actif, soit contiennent un fort pourcentage de glycol propénétrant afin de favoriser la pénétration de l'actif mais sont collants et peuvent provoquer des problèmes d'intolérance (voir article « The critical role of the vehicle to therapeutic efficacy and patient compliance », Piacquadio et al, J. Am. Acad. Dermatol, August 1998 »).

Les compositions de type onguent actuellement sur le marché ne se prêtent pas toujours à la formulation du principe actif sous une forme solubilisée.

L'un des buts de la présente invention est de proposer une composition pharmaceutique anhydre destinée à une application topique et permettant de s'affranchir des inconvénients précités.

Un autre but de la présente invention est de proposer une composition pharmaceutique anhydre destinée à une application topique dont les principes actifs sont sous une forme solubilisée et présentent une stabilité prolongée.

Un autre but de la présente invention est de proposer une composition pharmaceutique anhydre destinée à une application topique et qui présente une très bonne tolérance.

Ainsi la présente invention a pour objet une composition pharmaceutique anhydre destinée au traitement du psoriasis **caractérisée en ce qu**'elle comprend un onguent oléagineux comprenant au moins un beurre et à titre de principes actifs du calcitriol et du 17-propionate de clobétasol et un constituant choisi parmi la vaseline et/ou l'huile de paraffine, lesdits actifs étant chacun sous une forme solubilisée dans ladite composition.

Par forme solubilisée, on entend une dispersion à l'état moléculaire dans un liquide, aucune cristallisation de l'actif n'étant visible à l'oeil nu ni même au microscope optique en polarisation croisée.

Avantageusement, la quantité de calcitriol sous forme solubilisée dans la composition est l'invention est de 0,00001 à 5% en poids par rapport au poids total de la composition, de préférence de 0,0001 à 3% en poids et plus particulièrement de 0,0003 à 1% en poids.

La quantité de 17-propionate de clobétasol sous forme solubilisée est de 0,0001 à 3% en poids par rapport au poids total de la composition, de préférence de 0,00005 à 1% en poids, et plus particulièrement de 0,001 à 0,1% en poids.

La composition de l'invention est destinée à une application topique.

Les principes actifs entrant dans la composition de l'invention, à savoir le calcitriol et le 17-propionate de clobétasol, sont à l'état solubilisé afin de conférer aux compositions de l'invention de bonnes propriétés de libération/pénétration dans la peau de chacun desdits principes actifs, et cela allié à une cinétique plus avantageuse. On entend par « bonne capacité de libération/pénétration » une bonne distribution de la composition de l'invention et donc des principes actifs qu'elle contient, à travers le stratum cornéum de la peau ainsi qu'à travers les couches sous-cutanées comme l'épiderme et le derme.

Au sens de la présente invention et selon la pharmacopée américaine (« USP »), on entend par onguent une préparation semi-solide destinée à une application externe sur la peau ou les muqueuses. Les onguents ou pommades sont utilisés en voie topique pour de nombreuses applications, par exemple comme crèmes barrières, antiseptiques, émollients etc..Les onguents sont utilisés pour leur effet émollient, ils sont simples d'application et pénètrent facilement dans la peau.

Il existe communément cinq types d'onguents, différenciés sur la base de leur composition physique. Le type le plus commun d'onguent, qui est celui concerné dans la présente invention, est l'onguent de base oléagineuse, dénommé « onguent oléagineux » ; cet onguent est anhydre, hydrophobe et contient majoritairement de la vaseline et/ou des huiles de paraffine. Les autres constituants de l'onguent peuvent être :
- des huiles d'origine minérale, animale, végétale ou synthétique ;
- un agent épaississant tel que les cires : d'oxigine minérale, végétale et animale ;
- les beurres : beurre de cacao, beurre de Karité, beurre de coprah ;
- la lanoline et ses dérivés.

La vaseline est un mélange d'hydrocarbures aliphatiques à longues chaînes et est un excellent hydratant. En effet, ses propriétés occlusives permettent de bloquer la perte insensible en eau transcutanée et de piéger l'eau sous la surface de la peau, grâce à la formation d'une membrane occlusive inerte (« Effects of petrolatum on stratum corneum structure and function » Ghadially & all ; Journal of the American Academy of dermatology 1992 ; 26 :387-96). La vaseline accélère la recouvrance des propriétés normales de la barrière cutanée dans le cas de la peau lésée, comme par exemple dans la dermatite atopique ou le psoriasis. La vaseline seule, de par son émollience, est un bon agent hydratant et peut atténuer des plaques de psoriasis. De plus, la vaseline est inerte donc n'a aucune incompatibilité quel que soit le (ou les) principe(s) actif(s).

Les onguents à base de vaseline sont reconnus parmi les meilleurs systèmes de libération d'actif en terme d'efficacité. En effet, la peau sous occlusion devient plus perméable et les principes actifs peuvent mieux pénétrer. Il est également connu que l'efficacité des stéroïdes topiques peut être augmentée par l'application d'un film occlusif créé par l'onguent ("Hospital practice : current treatment options in psoriasis" Khachemoune et al, 2000, p.1-13.

Comme exemple d'huile minérale, on peut citer des huiles de paraffine de différentes viscosités telles que le Primol 352, le Marcol 82, Marcol 152 vendus par la société Esso.

Comme huile végétale, on peut citer l'huile d'amande douce, l'huile de palme, l'huile de soja, l'huile de sésame, l'huile de tournesol.

Comme huile animale, on peut citer la lanoline, le squalene, l'huile de poisson, l'huile de vison

Comme huile synthétique, on peut citer un ester tel que le cetearyl isononanoate comme le produit vendu sous le nom de Cetiol SN par la société Cognis France, le diisopropyl adipate comme le produit vendu sous le nom de Ceraphyl 230 par la société ISF, le palmitate d'isopropyle comme le produit vendu sous le nom de Crodamol IPP par la société Croda, le caprylique caprique triglyceride tel que Miglyol 812 vendu par la société Huls / Lambert Rivière.

Par « cire », on entend d'une manière générale un composé lipophile, solide à température ambiante (25 °C), à changement d'état solide / liquide réversible, ayant un point de fusion supérieur ou égal à 30 °C pouvant aller jusqu'à 200 °C et notamment jusqu'à 120 °C.

Les cires susceptibles d'être utilisées dans les compositions selon l'invention sont choisies dans le groupe constitué par les cires d'origine animale, végétale, minérale ou de synthèse et leurs mélanges.

Selon un mode de réalisation particulier, la cire hydrocarbonée peut être choisie parmi les esters de glycéryle et d'acides gras saturés et insaturés, notamment polyinsaturés ayant en particulier de 10 à 24 atomes de carbone, les acides gras insaturés et en particulier parmi les acides gras polyinsaturés.

Comme cires hydrocarbonées de type esters de glycérides et d'acides gras polyinsaturés pouvant être utilisées dans les compositions selon l'invention, on peut citer en particulier le dipalmitostéarate de glycéryle atomisé (C₁₆-C₁₈) commercialisé sous la dénomination de « Précirol ATO 5^{®} » par la société GATTEFOSSE, le béhénate de glycéryle atomisé (C₂₂) par exemple commercialisé sous la dénomination de « Compritol^{®} » par la société GATTEFOSSE, et leurs mélanges.

On peut également utiliser les cires hydrocarbonées comme la cire d'abeille, la cire de lanoline et les cires d'insectes de Chine ; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C₈-C₃₂.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50^{®}, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendu sous la dénomination HEST 2T-4B par la société HETERENE.

On peut encore citer les cires de silicone, les cires fluorées.

On peut également utiliser la cire obtenue par hydrogénation d'huile d'olive estérifiée avec l'alcool stéarylique vendue sous la dénomination «PHYTOWAX Olive 18 L 57» ou bien encore les cires obtenues par hydrogénation d'huile de ricin estérifiée avec l'alcool cétylique, vendues sous la dénomination « PHYTOWAX ricin 16L64 et 22L73 » par la société SOPHIM. De telles cires sont décrites dans la demande FR-A-2792190.

L'agent épaisissant peut être choisi parmi les beurres et la lanoline.

Selon un mode de réalisation préféré de l'invention, l'agent épaississant est la cire d'abeille, l'huile de ricin hydrogénée, la cire de carnauba, la cire alkyl méthyl siloxane (« ST wax 30 »), la cire de Candelilla.

La teneur en agent épaississant additionnel dépend bien entendu de la viscosité de la composition recherchée. Elle peut être bien entendu déterminée par l'homme de métier à l'aide de simples manipulations de routine.

De façon générale, la teneur en agent épaississant additionnel, et en particulier en composé hydrocarboné pâteux ou solide, est de 2 à 20 % en poids par rapport au poids total de la composition, en particulier de 5 à 10 %.

Le beurre de karité est une matière grasse végétale utilisée traditionnellement sur le continent africain, comme produit de base de la pharmacologie africaine. A titre d'exemple, les nouveaux-nés africains sont frottés dès leur naissance avec du beurre de Karité pour les protéger de conditions climatiques extrêmes. Il est utilisé par les chimistes cosméticiens depuis plus de 20 ans. C'est une graisse naturelle obtenue de l'arbre de Karité (Butyrospermum Parkii). L'analyse chimique du beurre de Karité révèle qu'il est composé d'acides gras et de leurs glycérides. Les acides gras présents dans le beurre de Karité sont saturés et non saturés. Le beurre de karité est notamment connu pour :
- ses vertus calmantes et adoucissantes sur les peaux sèches et sensibles,
- ses effets décongestionnants,
- son efficacité sur la diminution des signes du vieillissement,
- son action cicatrisante et régénératrice,
- son action hydratante,
- sa protection anti-érythémateuse UV (sur animaux).

En outre, la tolérance du beurre de karité est excellente.

Comme précisé ci-dessus, la teneur en beurre est de 2 à 20 % en poids par rapport au poids total de la composition, en particulier de 5 à 10 %.

Selon un mode de réalisation avantageux de l'invention, les composants de l'onguent oléagineux sont plus particulièrement choisis dans le groupe constitué par la vaseline et/ou l'huile de paraffine, le beurre de karité, et en outre, un émollient.

Un produit émollient a pour action de rendre la peau souple et lisse et de favoriser le bien être cutané. Il y a action émolliente soit par hydratation du stratum cornéum soit par compensation de l'insuffisance de la sécrétion sébacée.

L'hydratation du stratum cornéum peut se faire de plusieurs façons : utilisation de substances freinants la deshydratation grâce à un effet occlusif (corps gras : cires, huiles, alcools gras, huiles de silicone) ou utilisation d'agents humectants (polyols, glycerine, urée)

L'insuffisance de la sécrétion sébacée est quant à elle compensée par l'utilisation de produits lipidiques, comme les huiles.

Selon un autre mode de réalisation avantageux de l'invention, les principes actifs sont solubilisés dans un même solvant ou dans plusieurs solvants.

Le solvant de la présente invention est choisi parmi les composés pharmaceutiquement acceptables, c'est-à-dire les composés dont l'utilisation est en particulier compatible avec une application sur la peau, les muqueuses et/ou les fibres kératiniques. Il est généralement fluide, et en particulier liquide, à température ambiante et pression atmosphérique.

À titre d'agents solvants selon l'invention, on pourra notamment citer :
- le propylène glycol,
- les huiles telles que les triglycérides capriliques et capriques (Miglyol 812), le cétéraryl isononanoate (Cetiol SN)et les huiles végétales (huile d'amande douce, huile de sesame, huile de tounesol...) et leurs mélanges,
et leurs mélanges.

L'agent solvant est généralement présent dans les compositions de l'invention en une quantité d'une part suffisante pour procurer la solubilité requise des principes actifs à formuler, et d'autre part compatible avec la nécessité de préserver une stabilité chimique prolongée de ces mêmes principes actifs. En d'autres termes, l'agent solvant se doit d'être inerte chimiquement vis-à-vis des principes actifs.

Avantageusement, la quantité de solvant pour chacun des actifs dans une composition de l'invention est de 10 à 30% en poids par rapport au poids total de la composition, de préférence de 5 à 20% en poids.

L'agent solvant confère également un effet bénéfique sur le taux de pénétration dans la peau des principes actifs.

La composition selon l'invention peut comprendre en outre différents autres ingrédients. Le choix de ces ingrédients supplémentaires, de même que celui de leurs quantités respectives, est effectué de manière à ne pas porter préjudice aux propriétés attendues pour la composition. En d'autres termes, ces composés ne doivent pas affecter la stabilité chimique des principes actifs, ni leur solubilité.

La composition de l'invention peut en outre comprendre un agent anti-irritant lipophile. A titre d'exemple on pourra citer l'alpha-DL tocophérol acétate, l'huile de Mélaleuca à feuilles alternes, l'extrait de thé vert, l'extrait de calendula, le beurre de karité.

Selon un autre mode de réalisation avantageux, la composition de l'invention peut en outre comprendre un agent anti-oxydant choisi dans le groupe constitué par le butylhydroxytoluène (BHT), le butylhydroxyanisole (BHA), l'alpha-tocophérol DL, le propyl gallate.

Selon un mode de réalisation préféré, la composition de l'invention est une composition pharmaceutique anhydre de type onguent, comprenant de la vaseline et en tant qu'actifs du calcitriol et du 17-propionate de clobétasol sous forme solubilisée.

Une telle composition est stable physiquement et chimiquement, est efficace de manière synergique sur le psoriasis, et permet une libération optimisée des deux actifs tout en permettant une très bonne tolérance.

Outre les actifs et la vaseline, une composition préférée de l'invention comprend également du beurre de karité et un émollient tel que le caprilic/capric triglycerides ou le cetearyl isononanoate ou une huile végétale, et du propylene glycol.

L'association de la vaseline, du beurre de karité et d'un émollient permet de garantir la très bonne tolérance de la composition de l'invention, tout en permettant de restaurer la barrière cutanée altérée par la pathologie du psoriasis.

Les compositions de l'invention se révèlent particulièrement efficaces pour préserver une stabilité chimique satisfaisante des principes actifs sensibles à l'oxydation, à l'eau et aux milieux aqueux d'une manière générale. Par « stabilité chimique satisfaisante », on entend une composition qui, au cours d'une période d'au moins trois mois, respectivement à température ambiante et à 40°C :
- ne présente pas de modification substantielle de son aspect macroscopique,
- comprend une teneur en principes actifs d'au moins 80%, en particulier d'au moins 90% et plus particulièrement d'au moins 95% de la teneur pondérale initiale.

La présente invention concerne encore l'utilisation d'un onguent oléagineux pour la préparation d'une composition pharmaceutique anhydre destinée au traitement du psoriasis, ladite composition comprenant à titre de principes actifs du calcitriol et du 17-propionate de clobétasol, lesdits principes actifs étant chacun sous une forme solubilisée dans ladite composition.

L'invention concerne l'utilisation telle que définie ci-dessus dans laquelle la composition est telle que définie ci-dessus.

Les exemples ci-après illustrent l'invention, ils ne la limitent en aucune façon.

### Exemple 1 Etude de tolérance locale d'une composition de l'invention.

Une étude de tolérance a été menée sur le véhicule de formulation (sans actifs) de l'invention comparativement à un véhicule connu pour sa grande tolérance (Daivonex®).

Traitement : une application quotidienne du jour 1 au jour 6, de 20µl de composition est effectuée sur l'oreille droite de souris.

Méthode d'évaluation : observation clinique et mesure de l'épaisseur de l'oreille de souris du jour 2 au jour 12. Pesée des animaux le jour 1 et le jour 12.

### Conclusion :

Après 12 jours le véhicule de l'invention s'est montrée non irritante et comparable au véhicule de Daivonex® et à celui du groupe non traité.

### Exemple 2 Solubilité et stabilité des compositions de l'invention

La stabilité du calcitriol a été évaluée dans les deux solvants huiles suivants : Miglyol 812 et Cetiol SN.

### a) Stabilité du calcitriol dans le Miglyol 812 (triglycérides capriliques et capriques)

On prépare une solution de calcitriol 30ppm dans qsp 100% de Miglyol 812 en présence de 0.4% de BHT (anti-oxydant), gardé sous azote.

Les résultats de technique de dosage par HPLC contre une référence sont indiqués dans le tableau 1 ci-dessous.

Le temps initial (T0) est considéré à 100%

**Tableau 1 :**

| Conditions de stabilité | T2 semaines | T4 semaines |
|---|---|---|
| +4°C | 98.2% | 105.2% |
| température ambiante | 95.8% | 98.0% |
| +40°C | 93.1% | 95.0% |

Le calcitriol est stable 1 mois en solution dans le caprilic/capric triglycéride.

### b) Stabilité du calcitriol dans le Cetiol SN (cétéaryl isononanoate)

Une solution de calcitriol 30ppm dans qsp 100% de Cetiol SN est préparée en présence de 0.4% de BHT, et conservé sous azote.

Les résultats de technique de dosage par HPLC contre une référence sont indiqués dans le tableau 2 ci-dessous.

Le temps initial (T0) est considéré à 100%

**Tableau 2 :**

| Conditions de stabilité | T2semaines | T4semaines |
|---|---|---|
| +4°C | 103.7% | 97.9% |
| TA | 99.6% | 98.7% |
| +40°C | 99.4% | 98.1% |

Le calcitriol est stable 1 mois en solution dans le cetearyl isononanoate.

### Exemple 3 : Préparation d'une composition de l'invention.

L'invention se rapporte à une formulation anhydre qui permet d'incorporer tous les constituants à température élevée pour laquelle la vaseline est liquide, et ainsi permettre un bon mélange des constituants. Ceci permet également d'obtenir une bonne stabilité à 30°C, sans exsudat.

Le procédé se réalise dans un bain-marie qui permet de maintenir une température homogène au cours de la préparation ; en outre, il est également important de couvrir le bêcher formulaire de manière à éviter un phénomène de croûtage. Le procédé est effectué à l'aide d'une pâle papillon qui permet une bonne circulation au sein de produits pâteux, assurant ainsi une bonne homogénéisation.

### a) Première étape : préparation de la phase grasse comprenant l'onguent oléagineux.

Dans un bêcher, on pèse la vaseline (onguent oléagineux), l'épaississant et l'anti-irritant lipophile

On chauffe à 75°C au bain-marie, sous faible agitation Rayneri (pâle papillon).

### b) Seconde étape : préparation de la phase comprenant les principes actifs.

On prépare une solution mère de calcitriol dans le solvant adéquat, on ajoute un antioxydant et on agite jusqu'à solubilisation de l'actif.

On pèse le 17-propionate de clobétasol et son solvant, et on agite jusqu'à solubilisation de l'actif.

Ces deux phases actives sont incorporées à la phase grasse telle que précédemment préparée, à 75°C sous agitation Rayneri.

On homogénéise et on laisse refroidir la composition jusqu'à 30°C au bain-marie sous agitation Rayneri.

Le conditionnement est réalisé à 30°C, température pour laquelle la composition n'a pas encore totalement repris en masse.

### Exemple 4 : exemples de compositions conformes à l'invention

**(1) Tableau 3**

| Composition 1 | |
|---|---|
| Matières premières | Quantités en % poids pour poids |
| WHITE PETROLATUM | Qs 100 |
| CARNAUBA WAX | 9 |
| CAPRYLIC/CAPRIC TRIGLYCERIDE | 10 |
| SHEA BUTTER | 2 |
| dl-ALPHA TOCOPHEROL ACETATE | 1 |
| CALCITRIOL | 0.0009 |
| CLOBETASOL PROPIONATE | 0.01 |
| PROPYLENE GLYCOL | 10 |

**(2) Tableau 4**

| Composition 2 | |
|---|---|
| Matières premières | Quantités en % poids pour poids |
| WHITE PETROLATUM | Qs 100 |
| BEEWAX | 5 |
| CAPRYLIC/CAPRIC TRIGLYCERIDE | 15 |
| SHEA BUTTER | 5 |
| BUTYLHYDROXYTOLUENE | 0.04 |
| CALCITRIOL | 0.0003 |
| CLOBETASOL PROPIONATE | 0.025 |
| PROPYLENE GLYCOL | 10 |

La stabilité physique des compositions 1 et 2 est mesurée par une observation macroscopique et microscopique de la composition à température ambiante, à 4°C et à 30°C après 15 jours, 1 mois, 2 mois et 3 mois.

A température ambiante, l'observation macroscopique permet de garantir l'intégrité physique des produits et l'observation microscopique permet de vérifier qu'il n'y a pas recristallisation des actifs solubilisés.

A 4°C, l'observation microscopique vérifie la non-recristallisation des actifs solubilisés.

A 30°C, l'observation macroscopique vérifie l'intégrité de chacune des compositions finales.

On complète la caractérisation de chacune des compositions finales par une mesure du seuil d'écoulement. On utilise un rhéomètre HAAKE de type VT550 avec un mobile de mesure SVDIN. Les rhéogrammes sont réalisés à 25°C et à la vitesse de cisaillement de 4 s⁻¹ (γ), et en mesurant la contrainte de cisaillement. Par seuil d'écoulement (τ0 exprimé en Pascal) on entend la force nécessaire (contrainte de cisaillement minimum) pour vaincre les forces de cohésion de type Van der Waals et provoquer l'écoulement. Le seuil d'écoulement est assimilé à la valeur trouvée à la vitesse de cisaillement de 4s-1.

Ces mesures sont réalisées à T0, après 1 mois, 2 mois et 3 mois.

### COMPOSITION 2

| **SPECIFICATIONS A T0** | | | | |
|---|---|---|---|---|
| **Tau 0** | | 219 | **Aspect macroscopique** | Onguent jaune pâle brillant. |
| **Centrifugation** | **3000tr/min** | RAS | **Aspect microscopique** | Nombreuses réfringences jaunes, violettes et bleues caractéristiques du réseau de vaseline.. |
| | **10000tr/min** | Exsudat | | |

### STABILITE PHYSIQUE

| | | | **T15 J** |
|---|---|---|---|
| **TA** | **Macroscopie** | | CONFORME |
| | **Microscopie** | | CONFORME |
| | **Tau 0** | | 230 |
| | **Centri** | 3000 | CONFORME |
| | | 10000 | CONFORME |
| **4°C** | **Macroscopie** | | CONFORME |
| | **Microscopie** | | CONFORME |
| **30°C** | **Macroscopie** | | CONFORME |
| | **Microscopie** | | CONFORME |
| | **Tau 0** | | NR |

### STABILITE CHIMIQUE

| | | | |
|---|---|---|---|
| **STABILITE Chimique** | **T0** | **TA** | **40°C** |
| | | **CALCITRIOL: 97.6%** | **CALCITRIOL : /** |
| | | **CLOBETASOL PROPIONATE : 97.3%** | **CLOBETASOL PROPIONATE : /** |
| | **T15j** | **TA** | **40°C** |
| | | **CALCITRIOL:95.1** | **CALCITRIOL:86.8** |
| | | **CLOBETASOL PROPIONATE : 95.2** | **CLOBETASOL PROPIONATE : 92.3** |

### Exemple 5 : Etude de la libération / pénétration in vitro sur peau humaine de l'actif 17-propionate de clobétasol contenu dans 3 formulations différentes dont une selon l'invention.

L'objectif est de quantifier la pénétration cutanée de l'actif formulé dans différentes formulations *in vitro* sur peau humaine après 16 heures d'application.

Formulations testées:
- Crème émolliente Temovate^{®} à 0.05 % (w/w) de 17-propionate de clobétasol
- Crème Temovate^{®} à 0.05 % (w/w) de 17-propionate de clobétasol
- Composition selon l'invention de composition suivante A :

| **Matières premières** | **Quantités en % poids pour poids** |
|---|---|
| WHITE PETROLATUM | Qs 100 |
| BEEWAX | 5 |
| CAPRYLIC/CAPRIC TRIGLYCERIDE | 15 |
| SHEA BUTTER | 5 |
| BUTYLHYDROXYTOLUENE | 0.04 |
| CALCITRIOL | 0.0003 |
| CLOBETASOL PROPIONATE | 0.05 |
| PROPYLENE GLYCOL | 10 |

La crème émolliente Temovate® est commercialisée par la société GLAXOSMITHKLINE.

**Conditions expérimentales :** L'absorption percutanée est évaluée grâce à des cellules de diffusion constituées de 2 compartiments séparés par la peau humaine. Les formulations ont été appliquées sans occlusion pendant un temps d'application de 16 heures. Les formulations ont été appliquées à raison de 10 mg de formulation par cm² (*i.e*. 10 microgrammes de clobétasol 17-propionate). Pendant la durée de l'étude, le derme est en contact avec un liquide récepteur non renouvelé en fonction du temps (mode statique). Les expériences ont été réalisées avec 3 échantillons de peau provenant de 3 donneurs différents. A la fin de la période d'application, l'excès de surface est enlevé et la distribution du 17-propionate de clobétasol est quantifiée dans les différents compartiments de la peau et dans le liquide récepteur. Les concentrations de 17-propionate de clobétasol ont été quantifiées en utilisant un méthode d'HPLC/MS/MS classiquement connu de l'homme de l'art. (LQ: 1 ng.mL⁻¹).

Les résultats sont exprimés en % de la dose appliquée (moyenne +/- écart-type) et sont consignés dans le tableau ci-dessous.

| **Formulation** | | **Quantité totale ayant pénétré** |
|---|---|---|
| Temovate crème émolliente | Mean | 5.00 |
| | SEM | 1.34 |
| Temovate crème | Mean | 8.43 |
| | SEM | 0.79 |
| Composition A | Mean | 9.33 |
| | SEM | 0.97 |

Les résultats montrent que la quantité de clobétasol ayant pénétré avec la composition selon l'invention est équivalente à celle de la crème Temovate.

## Revendications

1. Composition pharmaceutique anhydre destinée au traitement du psoriasis **caractérisée en ce qu'**elle comprend un onguent oléagineux comprenant du beurre de karité, un constituant choisi dans le groupe constitué par la vaseline et/ou l'huile de paraffine, et en outre un émollient, et à titre de principes actifs du calcitriol et du 17-propionate de clobétasol, lesdits actifs étant chacun sous une forme solubilisée dans ladite composition, lesdits principes actifs sont solubilisés dans un même solvant ou dans plusieurs solvants choisis dans le groupe constitué par :
- le propylène glycol ;
- les huiles telles que les triglycérides capriliques et capriques, le cétéraryl isononanoate, les huiles végétales (huile d'amande douce, huile de sésame, huile de tournesol, et leurs mélanges ;
et leurs mélanges.

2. Composition selon la revendication 1, **caractérisée en ce qu'**elle est destinée à une application topique.

3. Composition selon l'une quelconque des revendications 1 à 2, **caractérisée en ce que** la quantité de calcitriol sous forme solubilisée est de 0,00001 à 5% en poids par rapport.au poids total de la composition, de préférence de 0,0001 à 3% en poids et plus particulièrement de 0,0003 à 1% en poids.

4. Composition selon l'une quelconque des revendications 1 à 2, **caractérisée en ce que** la quantité de 17-propionate de clobétasol sous forme solubilisée est de 0, 0001 à 3% en poids par rapport au poids total de la composition, de préférence de 0,00005 à 1% en poids, et plus particulièrement de 0,001 à 0,1% en poids.

5. Composition selon l'une quelconque des revendications 2 à 4, **caractérisée en ce que** la quantité de solvant est de 10 à 30% en poids-par rapport au poids total de la composition, de préférence de 5 à 20% en poids.

6. Composition selon l'une quelconque des revendications 1 à 5, **caractérisée en ce qu'**elle comprend en outre un agent épaississant choisi parmi les composés hydrocarbonés solides ou pâteux tels que les cires hydrocarbonées, d'origine animale, végétale, minérale, de synthèse, les beurres, la lanoline et leurs mélanges.

7. Composition selon l'une quelconque des revendications 1 à 6, **caractérisée en ce qu'**elle comprend en outre un agent anti-irritant lipophile.

8. Composition selon l'une quelconque des revendications 1 à 7, **caractérisée en ce qu'**elle comprend en outre un agent anti-oxydant.

9. Composition selon l'une quelconque des revendications 1 à 8, **caractérisée en ce qu'**elle comprend un ou plusieurs excipients pharmaceutiques adaptés pour une application topique.

10. Utilisation d'un onguent oléagineux selon la revendication 1 pour la préparation d'une composition anhydre comprenant du calcitriol solubilisé et du 17-propionate de clobétasol solubilisé.

11. Utilisation selon la revendication 10, dans laquelle la composition est telle que définie à l'une quelconque des revendications 1 à 9.

## Claims

1. Anhydrous pharmaceutical composition intended for the treatment of psoriasis, **characterized in that** it comprises an oleaginous ointment comprising karite butter, a component selected from the group consisting of petroleum jelly and/or liquid paraffin and also an emollient, and as active principles calcitriol and clobetasol 17-propionate, the said actives each being in a solubilized form in the said composition, the said active principles are solubilized in a single solvent or in two or more solvents selected from the group consisting of:
- propylene glycol,
- oils such as caprylic and capric triglycerides, cetearyl isononanoate, vegetable oils (sweet almond oil, sesame oil, sunflower oil) and mixtures thereof; and
- mixtures thereof.

2. Composition according to Claim 1, **characterized in that** it is intended for topical application.

3. Composition according to either of Claims 1 and 2, **characterized in that** the amount of calcitriol in solubilized form is from 0.00001 to 5% by weight relative to the total weight of the composition, preferably from 0.0001 to 3% by weight and more particularly from 0.0003 to 1% by weight.

4. Composition according to either of Claims 1 and 2, **characterized in that** the amount of clobetasol 17-propionate in solubilized form is from 0.0001 to 3% by weight relative to the total weight of the composition, preferably from 0.00005 to 1% by weight and more particularly from 0.001 to 0.1% by weight.

5. Composition according to any one of Claims 2 to 4, **characterized in that** the amount of solvent is from 10 to 30% by weight relative to the total weight of the composition, preferably from 5 to 20% by weight.

6. Composition according to any one of Claims 1 to 5, **characterized in that** it further comprises a thickener selected from solid or pasty hydrocarbon compounds such as hydrocarbon waxes of animal, vegetable, mineral or synthetic origin, butters, lanoline and mixtures thereof.

7. Composition according to any one of Claims 1 to 6, **characterized in that** it further comprises a lipophilic anti-irritant.

8. Composition according to any one of Claims 1 to 7, **characterized in that** it further comprises an antioxidant.

9. Composition according to any one of Claims 1 to 8, **characterized in that** it comprises one or more pharmaceutical excipients suitable for topical application.

10. Use of an oleaginous ointment according to Claim 1 for preparing an anhydrous composition comprising solubilized calcitriol and solubilized clobetasol 17-proprionate.

11. Use according to Claim 10, wherein the composition is as defined in any one of Claims 1 to 9.

## Patentansprüche

1. Wasserfreie pharmazeutische Zusammensetzung, die für die Behandlung von Psoriasis vorgesehen ist, **dadurch gekennzeichnet, dass** sie eine ölhaltige Salbe umfasst, die Sheabutter, einen Bestandteil, der unter Vaseline und/oder Paraffinöl ausgewählt ist, und ferner einen Geschmeidigmacher und als Wirkstoffe Calcitriol und Clobetasol-17-propionat enthält, wobei die Wirkstoffe jeder in der Zusammensetzung in gelöster Form vorliegen und wobei die Wirkstoffe in einem einzigen oder in mehreren Lösemitteln gelöst sind, die ausgewählt sind unter:
- Propylenglycol,
- Ölen, wie Triglyceriden von Capryl- und Caprinsäure, Cetearylisononanoat, pflanzlichen Ölen (Süßmandelöl, Sesamöl, Sonnenblumenöl und deren Gemischen;
- und deren Gemischen.

2. Zusammensetzung nach Anspruch 1, **dadurch gekennzeichnet, dass** sie für eine topische Anwendung vorgesehen ist.

3. Zusammensetzung nach einem der Ansprüche 1 bis 2, **dadurch gekennzeichnet, dass** der Mengenanteil des Calcitriols in solubilisierter Form 0,00001 bis 5 Gew.-%, bezogen auf das Gesamtgewicht der Zusammensetzung, vorzugsweise 0,0001 bis 3 Gew.-% und insbesondere 0,0003 bis 1 Gew.-% beträgt.

4. Zusammensetzung nach einem der Ansprüche 1 bis 2, **dadurch gekennzeichnet, dass** der Mengenanteil des Clobetasol-17-propionats in solubilisierter Form 0,0001 bis 3 Gew.-%, bezogen auf das Gesamtgewicht der Zusammensetzung, vorzugsweise 0,00005 bis 1 Gew.-% und insbesondere 0,001 bis 0,1 Gew.-% beträgt.

5. Zusammensetzung nach einem der Ansprüche 2 bis 4, **dadurch gekennzeichnet, dass** der Mengenanteil des Lösungsmittels im Bereich von 10 bis 30 Gew.-%, bezogen auf das Gesamtgewicht der Zusammensetzung, vorzugsweise im Bereich von 5 bis 20 Gew.-% liegt.

6. Zusammensetzung nach einem der Ansprüche 1 bis 5, **dadurch gekennzeichnet, dass** sie ferner ein Verdickungsmittel enthält, das unter den festen oder pastösen Kohlenwasserstoffverbindungen, wie Kohlenwasserstoffwachsen tierischer, pflanzlicher, mineralischer oder synthetischer Herkunft, Butterverbindungen, Lanolin, und deren Gemischen ausgewählt ist.

7. Zusammensetzung nach einem der Ansprüche 1 bis 6, **dadurch gekennzeichnet, dass** sie ferner einen lipophilen reizlindernden Wirkstoff enthält.

8. Zusammensetzung nach einem der Ansprüche 1 bis 7, **dadurch gekennzeichnet, dass** sie ferner ein Antioxidationsmittel enthält.

9. Zusammensetzung nach einem der Ansprüche 1 bis 8, **dadurch gekennzeichnet, dass** sie einen oder mehrere pharmazeutische Hilfsstoffe enthält, die für eine topische Anwendung geeignet sind.

10. Verwendung einer ölhaltigen Salbe nach Anspruch 1 für die Herstellung einer wasserfreien Zusammensetzung, die gelöstes Calcitriol und gelöstes Clobetasol-17-propionat enthält.

11. Verwendung nach Anspruch 10, wobei es sich um eine wie in einem der Ansprüche 1 bis 9 definierte Zusammensetzung handelt.
